(19) Europäisches Patentamt
European Patent Office
Office européen des brevets

(11) Numéro de publication: **0 141 690**
**A1**

(12) **DEMANDE DE BREVET EUROPEEN**

(21) Numéro de dépôt: **84401697.2**

(22) Date de dépôt: **21.08.84**

(51) Int. Cl.⁴: **C 07 B 61/02,** C 08 F 10/00, C 08 F 4/32 // C07C1/253, C07C9/22, C07C15/12, C07C17/33, C07C19/02, C07C21/24, C07C23/28, C07C29/00, C07C33/24, C07D213/70, C07J7/ 00, C07J9/00, C07J43/00

(30) Priorité: **23.08.83 FR 8313598**

(43) Date de publication de la demande: **15.05.85 Bulletin 85/20**

(84) Etats contractants désignés: **AT BE CH DE FR GB IT LI LU NL SE**

(71) Demandeur: **CENTRE NATIONAL DE LA RECHERCHE SCIENTIFIQUE (CNRS), 13 Quai Anatole France, F-75700 Paris (FR)**

(72) Inventeur: **Barton, Derek Harold Richard, Route de Chateaufort, F-91190 Gif Sur Yvette (FR)**
Inventeur: **Crich, David, 4, place de la Poste, F-91449 Bures (FR)**
Inventeur: **Motherwell, William Branks, 3, Résidence des Quinconces Route de Chateaufort, F-91190 Gif Sur Yvette (FR)**

(74) Mandataire: **Ahner, Francis et al, CABINET REGIMBEAU 26, avenue Kléber, F-75008 Paris (FR)**

(54) **Procédé de formation de radicaux carbonés libres, ses applications en particulier à la polymerisation radicalaire.**

(57) L'invention concerne un procédé de formation de radicaux carbonés libres R. éventuellement fonctionnalisés, caractérisé en ce que l'on apporte de l'énergie thermique et/ou photochimique à un ester thiocarbonylé répondant à la formule générale (I):

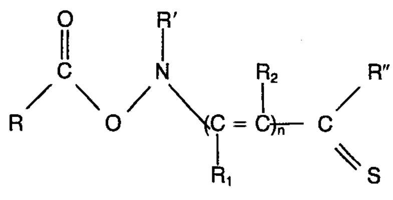

dans laquelle:

R représente un radical carboné saturé ou insaturé, linéaire ou ramifié, aliphatique ou aromatique, acyclique ou bien mono- ou poly-cyclique ou encore mono- ou poly-hétérocyclique, ledit radical carboné R étant en outre éventuellement fonctionnalisé;

R' et R'' représentent, indépendamment l'un de l'autre, un radical alcoyle, alcényle, aryle, aralcoyle, alcoylaryle, ou bien forment ensemble un hétérocycle azoté à 5 ou 6 chaînons pouvant en outre contenir un hétéroatome additionnel choisi parmi l'azote et le soufre et pouvant éventuellement être substitué ou accolé à au moins un autre cycle aliphatique ou aromatique;

$R_1$ et $R_2$ représentent, indépendamment l'un de l'autre, un atome d'hydrogène ou un radical alcoyle inférieur, et

n est un nombre entier égal à 0, 1, 2 ou 3.

EP 0 141 690 A1

# PROCEDE DE FORMATION DE RADICAUX CARBONES LIBRES, SES APPLICATIONS EN PARTICULIER A LA POLYMERISATION RADICALAIRE.

La présente invention concerne un procédé de formation de radicaux carbonés libres R. éventuellement fonctionnalisés et portant leur électron libre sur un atome de carbone.

Au cours des dernières années, les réactions radicalaires ont connu un grand essor dans le cadre général de la synthèse organique. Ces réactions radicalaires présentent en effet un certain nombre d'avantages importants par rapport aux réactions ioniques plus classiques. Tout d'abord, les réactions radicalaires en chaîne peuvent généralement-être conduites dans des conditions neutres. Ces réactions s'effectuent, en outre, dans des conditions très douces, ce qui permet d'éviter les interférences de nature stérique ou polaire sur les produits de départ. Au surplus, ce type de réactions ne s'accompagne généralement pas de réactions parasites de réarrangement carbocationique ou d'élimination carbanionique.

La présente invention a donc eu pour but de mettre au point un nouveau procédé de formation de radicaux carbonés libres dont la fonctionnalité ne se trouve pas modifiée par rapport aux produits de départ. Le procédé de l'invention consiste essentiellement en une décarboxylation radicalaire d'esters d'acides organiques aussi bien primaires, secondaires que tertiaires.

Les conditions douces de mise en oeuvre de ce procédé ont permis l'obtention, avec d'excellents rendements, de radicaux libres conservant notamment les fonctions ester, cétone et oléfine des produits de départ.

Conformément à la présente invention, les radicaux carbonés libres R. éventuellement fonctionnalisés sont obtenus par apport d'énergie thermique et/ou photochimique à un ester thiocarbonylé répondant à la formule générale (I) :

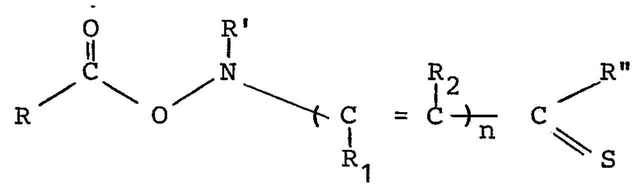

(I)

dans laquelle :

R représente un radical carboné saturé ou insaturé, linéaire ou ramifié, aliphatique ou aromatique, acyclique ou bien mono- ou poly-cyclique ou encore mono- ou poly-hétérocyclique, ledit radical carboné R étant en outre éventuellement fonctionnalisé ;

R' et R" représentent, indépendamment l'un de l'autre, un radical alcoyle, alcényle, aryle, aralcoyle, alcoylaryle, ou bien forment ensemble un hétérocycle azoté à 5 ou 6 chaînons pouvant en outre contenir un hétéroatome additionnel choisi parmi l'azote et le soufre et pouvant éventuellement être substitué ou accolé à au moins un autre cycle aliphatique ou aromatique ;

$R_1$ et $R_2$ représentent, indépendamment l'un de l'autre, un atome d'hydrogène ou un radical alcoyle inférieur, et

n est un nombre entier égal à 0, 1, 2 ou 3.

Selon une caractéristique additionnelle de la présente invention, ledit ester thiocarbonylé est porté à une température sensiblement comprise entre 20 et 200°C, de préférence entre 70 et 120°C.

L'apport d'énergie photochimique est généralement nécessaire lorsque l'on opère dans des gammes de faibles températures, par exemple inférieures à environ 40°C. Quelle que soit la température utilisée, l'apport d'énergie photochimique additionnelle conduit à une élévation de la vitesse de réaction. Cette énergie photochimique peut être apportée au milieu réactionnel contenant ledit ester thiocarbonylé par irradiation lumineuse, notamment de lumière visible.

A titre d'illustration des esters thiocarbonylés de formule générale (I) utilisables dans le cadre de la présente invention, on mentionnera les exemples non limitatifs suivants correspondant aux formules suivantes :

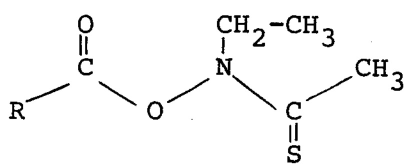

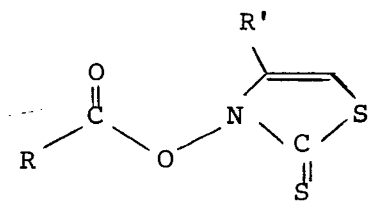

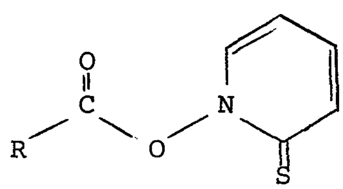

(II)

où R et R' ont les significations données précédemment à propos de la formule générale (I) et R' est de préférence Me, H ou Ph par exemple.

Ces différents esters thiocarbonylés peuvent être préparés par des procédés de synthèse classiques. Dans le cadre de la présente invention, les esters thionopyridoniques de formule générale (II) peuvent

toutefois être préparés avantageusement par réaction d'un acide carboxylique libre de formule générale (III) :

$RCO_2H$ (III)

dans laquelle R a la signification donnée à propos de la formule (I), avec un composé de formule (IV) :

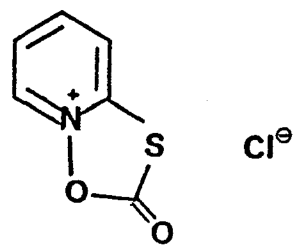

(IV)

ce dernier étant obtenu par réaction du phosgène sur la N-hydroxy-pyridine-2-thione.

L'ester thionopyridonique de formule (II) peut également être préparé dans d'excellentes conditions par réaction de l'anhydride mixte de formule générale (V)

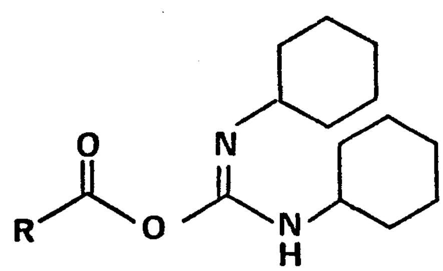

(V)

dans laquelle R a la signification donnée précédemment, avec la N-hydroxy-pyridine-2-thione en présence d'un catalyseur tel que la p-diméthylamino-pyridine.

Enfin, l'ester thionopyridonique de formule (II) peut être obtenu par réaction d'un chlorure d'acide de formule générale (VI) :

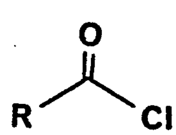

(VI)

dans laquelle R a la signification donnée à propos de la formule (I), avec le sel de sodium de la N-hydroxy-pyridine-2-thione, en présence d'un catalyseur tel que la p-diméthylaminopyridine.

Ces trois modes de préparation sont résumés sur le schéma réactionnel indiqué ci-après :

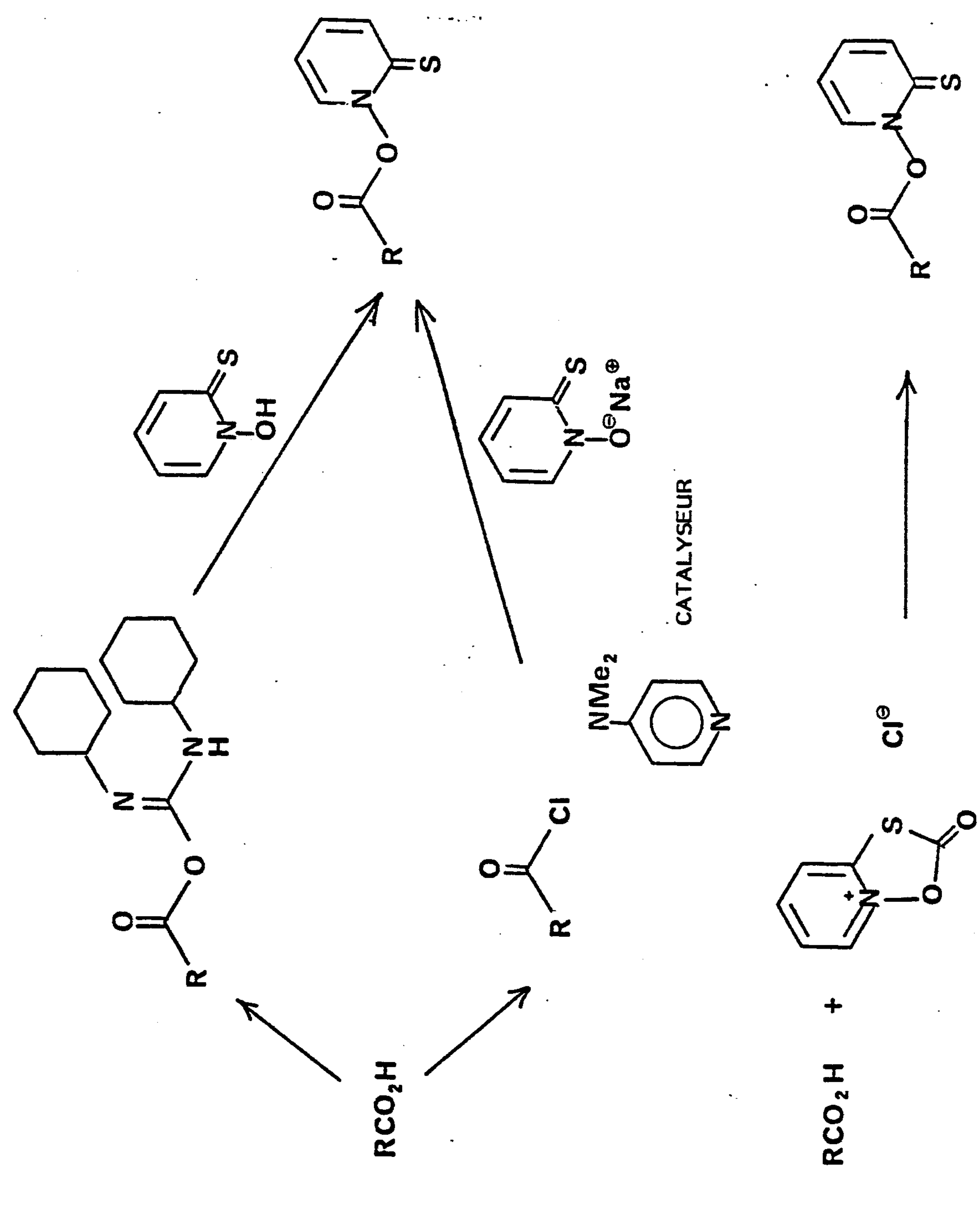
CATALYSEUR

La présente invention s'étend également à la préparation d'un certain nombre de dérivés décarboxylés de formules générales R—X ou R—A—B—X.

La présente invention concerne en particulier un procédé de préparation d'un composé de formule générale R—X à partir d'un acide carboxylique de formule générale R—$CO_2H$, dans lesquelles R a la signification donnée à propos de la formule (I), procédé caractérisé en ce que l'on ajoute au milieu réactionnel de formation de radicaux libres carbonés R. un composé de formule générale X—Y dans laquelle X, qui représente le groupe à greffer sur le radical libre carboné R., est choisi parmi les atomes d'hydrogène, de chlore, de brome et d'iode,etc.,et Y représente un groupe porteur de chaîne choisi parmi n-$Bu_3$ S$^{\cdot}$, tert-Bu S$^{\cdot}$, $CCl_3^{\cdot}$, $CHI_2^{\cdot}$, $R_3Sn^{\cdot}$, $ArSO_2^{\cdot}$, etc.

L'invention concerne également un procédé de préparation d'un composé de formule générale R—A—B—X à partir d'un acide carboxylique de formule générale R—$CO_2$—H, dans lesquelles R a la signification donnée pour la formule (I), procédé caractérisé en ce que, en plus du composé de formule X—Y défini précédemment, on ajoute au milieu réactionnel de formation de radicaux libres carbonés R. un composé de formule générale A═B choisi parmi l'oxygène, les composés à insaturation éthylénique et les dérivés azoïques.

Il convient de noter que, par réaction avec un seul monomère éthylénique A═B, le procédé de l'invention conduit à un simple allongement de la chaîne carbonée de l'acide de départ avec une décarboxylation concomitante. En présence de plusieurs monomères, le procédé trouve son application dans la polymérisation radicalaire, en particulier éthylénique. Etant donné que les esters thiocarbonylés de formules (I) et (II) sont entièrement solubles dans les alcalis, ils peuvent être

éliminés sans aucune difficulté lorsqu'ils sont présents en excès dans le milieu réactionnel de polymérisation.

Le processus général de formation des radicaux carbonés libres R. est schématisé ci-après à propos de l'ester thionopyridonique particulier de formule (II), afin d'en faciliter la compréhension :

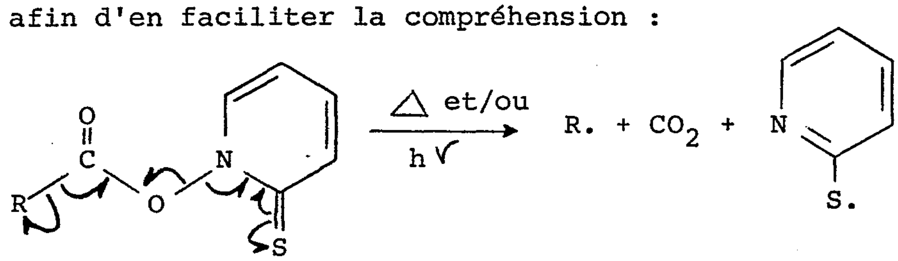


Schéma 1

Le processus reste bien sûr le même dans le cas de l'ester thiocarbonylé de formule (I), par transfert le long de chaîne oléfinique.

Comme cela a été mentionné précédemment, l'ester thiocarbonylé formateur de radicaux libres R. est obtenu à partir d'acides carboxyliques primaires, secondaires ou ternaires. On indiquera ci-après, à titre d'illustration, quelques exemples d'acides qui ont été utilisés dans la pratique. Ces acides sont mentionnés ci-après par leur formule générale et sont suivis de divers dérivés obtenus dans le cadre de la présente invention. Ces composés sont repérés par un nombre entre parenthèses, qui sera conservé pour les identifier dans le reste de la description.

<u>ACIDES PRIMAIRES</u> ($X = CO_2H$)

$$CH_3(CH_2)_nX$$

(1) n = 14 ; X = $CO_2H$

(2) n = 14 ; X = H

(3) n = 14 ; X = 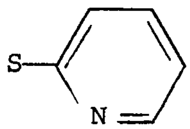

(4) n = 14 ; X = Cl

(5) n = 14 ; X = Br

(6) n = 14 ; X = I

(7) n = 16 ; X = $CO_2H$

(8) n = 16 ; X = H

(9) n = 16 ; X = 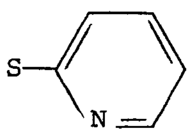

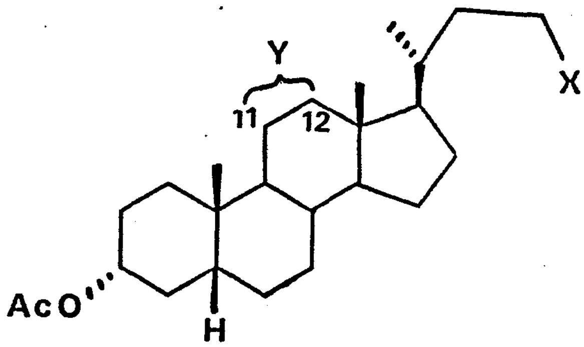

(10) Y = 12-oxo ; X = $CO_2H$
(11) Y = 12-oxo ; X = H
(12) Y = 11-oxo ; X = $CO_2H$
(13) Y = 11-oxo ; X = H
(14) Y = 11-oxo ; X = S 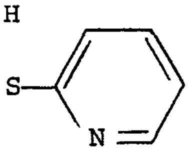
(15) Y = 11-oxo ; X = Br
(16) Y = 12-(α)-acétoxy ; X = $CO_2H$
(17) Y = 12-(α)-acétoxy ; X = H
(18) Y = 12-(α)-acétoxy ; X = S 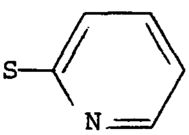
(19) Y = 12-(α)-acétoxy ; X = Cl

ACIDES SECONDAIRES (X = $CO_2H$)

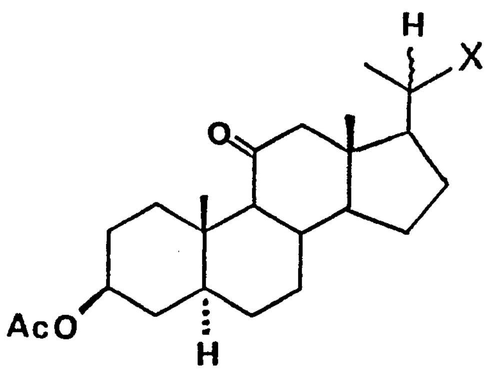

(20) X = $CO_2H$

(21) X = H

(22) X = S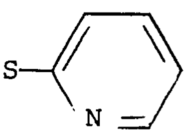

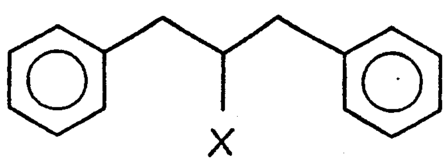

(23) X = $CO_2H$

(24) X = H

(25) X = S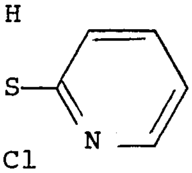

(26) X = Cl

(27) X = Br

(28) X = I

<u>ACIDES TERTIAIRES</u> ($X = CO_2H$)

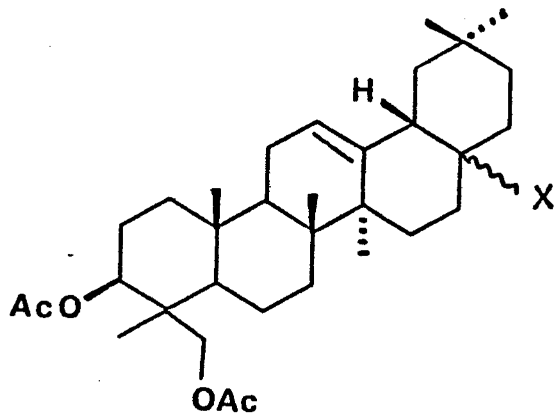

(<u>29</u>) X = (β)-$CO_2H$

(<u>30</u>) X = H

(<u>31</u>) X = 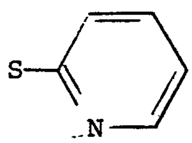

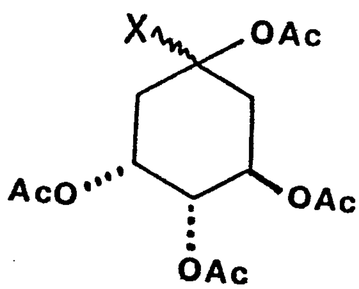

(<u>32</u>) X = (β)-$CO_2H$

(<u>33</u>) X = H

(<u>34</u>) X = 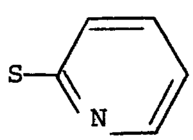

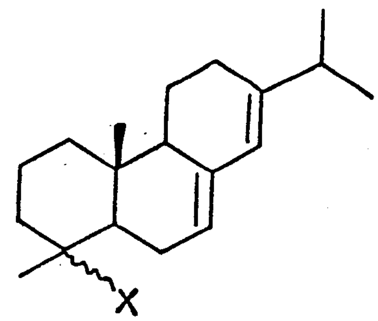

(35) X = (α)-$CO_2H$

(36) X = H

(37) X = 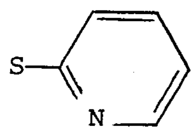

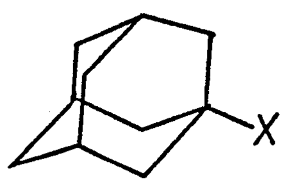

(38) X = $CO_2H$

(39) X = H

(40) X = 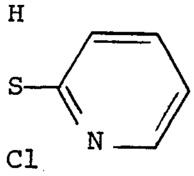

(41) X = Cl

(42) X = Br

Le procédé objet de la présente invention sera décrit ci-après plus en détail en référence à quelques exemples particuliers de mise en oeuvre, qui ont été regroupés par type d'applications particulières et dont on indiquera à chaque fois le schéma du mécanisme réactionnel.

EXEMPLES 1 à 11

L'apport d'énergie thermique et/ou photochimique à l'ester thionopyridonique de formule générale (II) conduit à la formation de radicaux carbonés libres selon le schéma 1 rappelé ci-après :

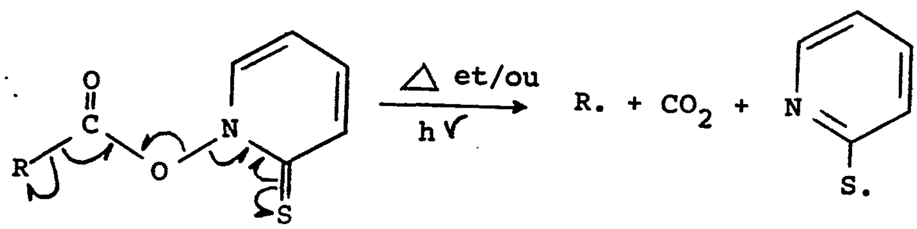


**Schéma 1**

Il se produit ensuite, toujours par suite de l'apport d'énergie, un réarrangement décarboxylatif de la N-acyloxy-2-pyridone de formule (II), conformément au schéma 2 ci-après :

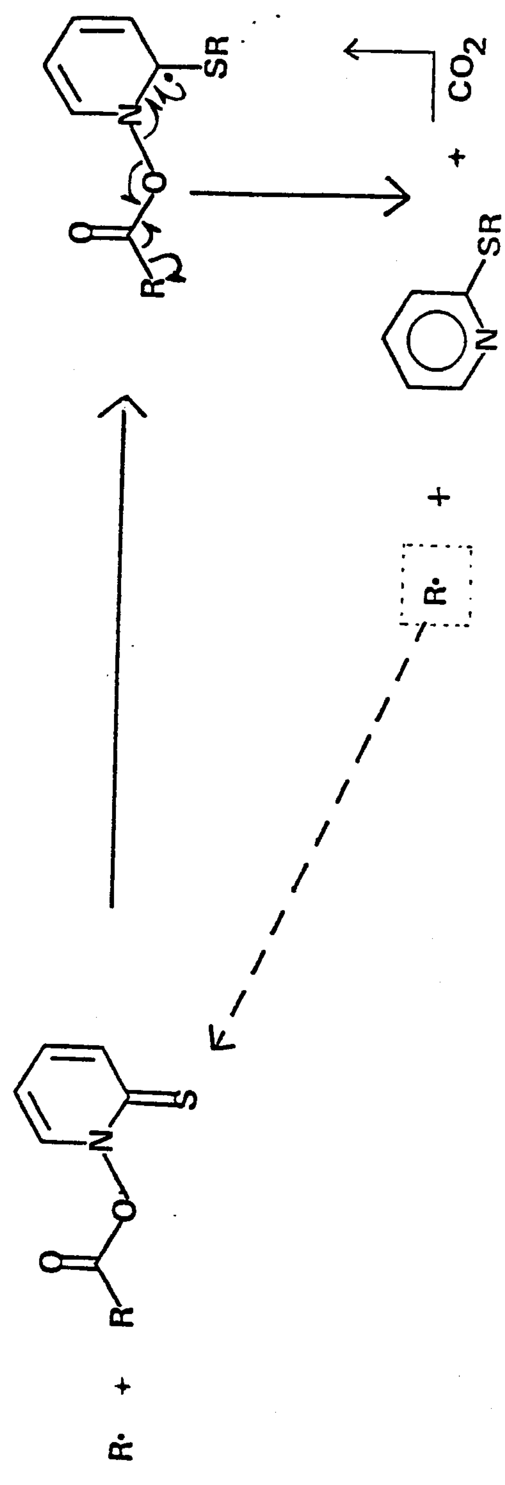

Schéma 2

Cette réaction radicalaire en chaîne conduit à la formation du pyridyle sulfure correspondant.

Exemple 1

Préparation de 1,3,4,5-tétraacétoxy-1-mercapto-(2'-pyridine)-cyclohexane

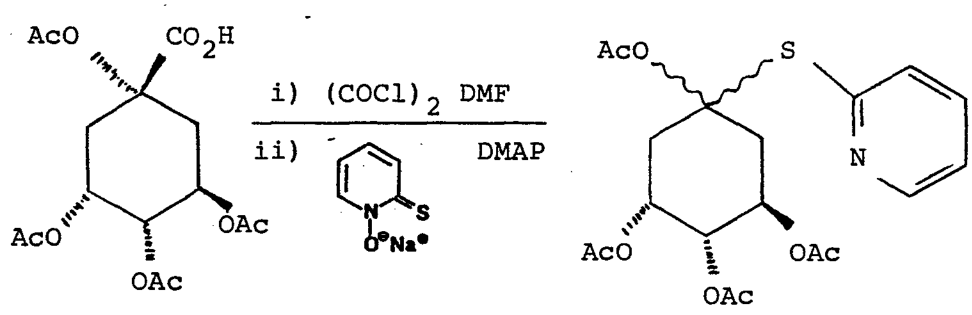


(32) (34)

360 mg (1 mmole) d'acide peracétoquinique (32) sont mis en solution sous agitation dans 5 ml de benzène avec addition de 1 ml de chlorure d'oxalyle et 1 goutte de DMF pendant 3 heures. Après évaporation à siccité, le résidu est repris dans 5 ml de benzène et additionné à une suspension sous agitation de 180 mg (1,1 mmole) du sel sodique de la N-hydroxy-pyridine-2-thione et de 12 mg (0,1 mmole) de DMAP. Le mélange réactionnel est maintenu au reflux dans 10 ml de toluène sous atmosphère d'azote. Après 2 heures de chauffage au reflux, le mélange réactionnel refroidi est filtré sur célite et évaporé à siccité. Par flash chromatographie sur silice (90 % $CH_2Cl_2$ ; 10 % EtOAc) on obtient 306 mg de pyridyle sulfure de formule (34) sous forme d'une huile jaunâtre. Rendement 72 %.

δ (400 MHz $CDCl_3$)

δ 2,00-2,13, 8 singulets 3H séparés.

2,30 (1HM) ; 2,41 (2HM) ; 2,50 (2HM) ;
2,80 (1HM) ; 3,22 (1HM) ; 3,40 (1HM) ;
5,00 (1HM) ; 5,18 (1HM) ; 5,25 (1HM) ;
5,28 (1HM) ; 5,36 (1HM) ; 5,57 (1HM) ;
7,25 (2HM) ; 7,43 (2HM) ; 7,62 (2HM) ;
8,55 (2HM).

ν ($CH_2Cl_2$) $cm^{-1}$ 1700, 1720-1740 large

M/Z 425 ($M^{+\cdot 7}$).

$\lambda_{max}$ nm 250 (4300), 284 (3600).

$[\alpha]_D^{18}$ - 100° (C=1 dans $CHCl_3$)

$C_{19}H_{23}NO_8S$

trouvé : C 53,89 ; H 5,71 ; N 3,15 ; S 7,41 %
calculé : C 53,64 ; H 5,44 ; N 3,29 ; S 7,54 %.

Exemple 2

En opérant de la même manière qu'à l'exemple 1, on réalise la réaction suivante :

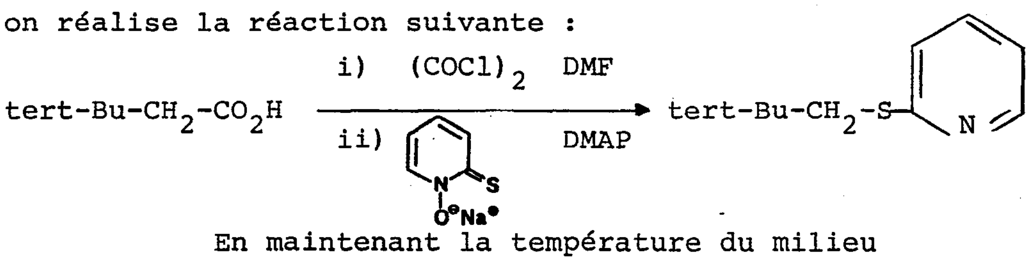


En maintenant la température du milieu réactionnel à 130°C pendant 1 heure et demie, on obtient le pyridyle sulfure avec un rendement de 78 %.

Exemple 3

En opérant de la même manière qu'à l'exemple 1, on réalise la réaction suivante :

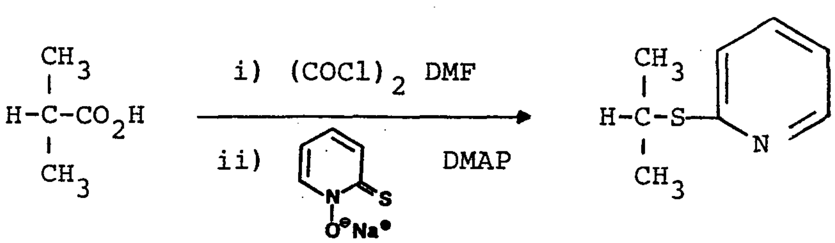

En maintenant la température du milieu réactionnel à 80°C pendant 2 heures et demie, on obtient le pyridyle sulfure avec un rendement de 78 %.

Exemple 4

En opérant de la même manière qu'à l'exemple 1, on réalise la réaction suivante :

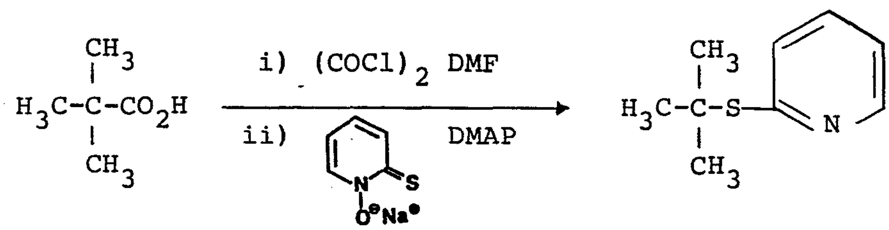


En maintenant la température du milieu réactionnel à 80°C pendant 1 heure et demie, on obtient le pyridyle sulfure avec un rendement de 71 %.

Exemple 5

Préparation du 3-(α)-acétoxy-11-oxo-23-mercapto-(2'-pyridine)-24-norcholane

432 g (1 mmole) d'acide acétyl-11-oxo-lithocholique sont additionnés dans 5 ml de benzène avec 1 ml de chlorure d'oxalyle et 1 goutte de DMF, et maintenus sous agitation pendant 3 heures. Après évaporation à siccité, le résidu est repris dans 5 ml de toluène et ajouté à une suspension sous agitation de 180 mg (1,1 mmole) du sel sodique de la N-hydroxy-pyridine-2-thione et de 12 mg (0,1 mmole) de DMAP. Le mélange réactionnel est maintenu au reflux dans 10 ml de toluène sous atmosphère d'azote. Après 1 heure et demie de chauffage au reflux, le mélange réactionnel refroidi est filtré et évaporé à siccité. Par flash chromatographie sur silice (100 % $CH_2Cl_2$), on obtient 382 mg du pyridyle sulfure de formule (14) sous forme d'une huile jaune qui ne cristallise pas. Rendement 77 %.

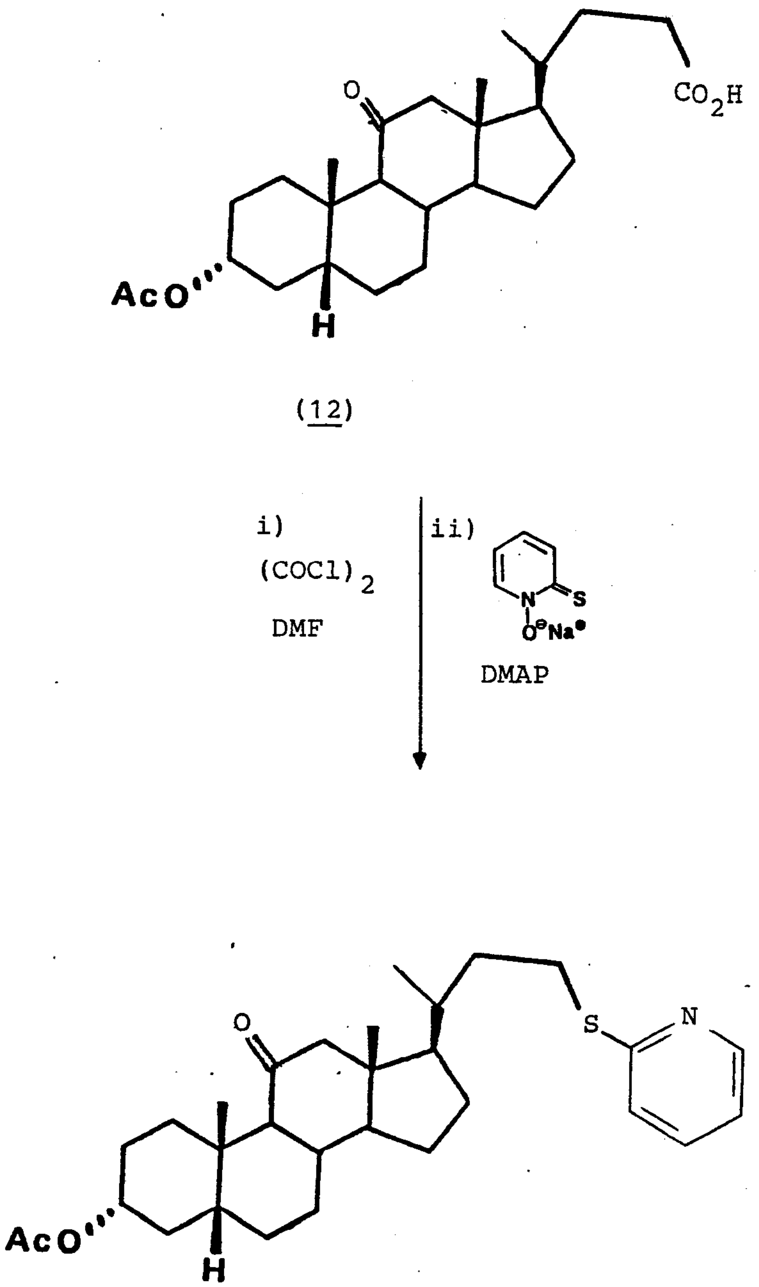


(12)

(14)

$\delta$ (400 MHz $CDCl_3$) o,65 (3H s) ; 1,00 (3H d, J = 7 Hz) ; 1,18 (3H s) ; 2,03 (3H s) ; 2,28 (1H d, J = 10 Hz) ; 2,40 (1H d, J = 10 Hz) ; 2,55 (2H m) ; 3,06 (1H m) ; 3,30 (1H m) ; 4,72 (1H m) ; 6,95 (1H dd, $J_1$ = 9 Hz, $J_2$ = 6 Hz) ; 7,15 (1H d, $J_3$ = 10 Hz) ; 7,37 (1H dd, $J_3$ = 10 Hz, $J_1$ = 9 HZ) ; 8,40 (1H d, $J_2$ = 6 Hz).

$\nu$ ($CH_3Cl_2$) $cm^{-1}$ 1700, 1720, 1575, 1550.

M/Z 497 ($M^{+\cdot 7}$).

$[\alpha]_D^{18}$ + 60° (C=0,9 dans $CHCl_3$).

$\lambda_{max}$ (EtOH) 253 nm (7980) ; 292 nm (2900).

$C_{23}\ H_{43}\ N\ O_2\ S$

trouvé : C 72,02 ; H 8,58 ; N 2,58

calculé : C 72,39 ; H 8,71 ; N 2,81.

En opérant de la même manière qu'à l'exemple 5 ci-dessus, on réalise un certain nombre de réactions du même type sur différents autres acides. Les conditions expérimentales, le rendement et la nature des produits sont consignés dans le tableau ci-après :

| Exemple | Acide | Température (°C) | Durée (heure) | Produit obtenu | Rendement (%) |
|---|---|---|---|---|---|
| n° 6 | (1) | 80 | 2 | (3) | 92 |
| n° 7 | (7) | 110 | 2 | (9) | 74 |
| n° 8 | (16) | 110 | 2,5 | (18) | 74 |
| n° 9 | (20) | 110 | 1,5 | (22) | 98 |
| n° 10 | (29) | 80 | 1 | (31) | 74 |
| n° 11 | (35) | 80 | 3 | (37) | 71 |

Les différents pyridyle sulfures ainsi obtenus sont en outre utiles comme intermédiaires de synthèse, comme décrit par exemple par Mukaiyama et coll. dans Chem. Letts., 1159 (1975) et Chem. Letts., 259 (1972).

Les diverses applications des pyridyle sulfures peuvent être schématisées comme suit.

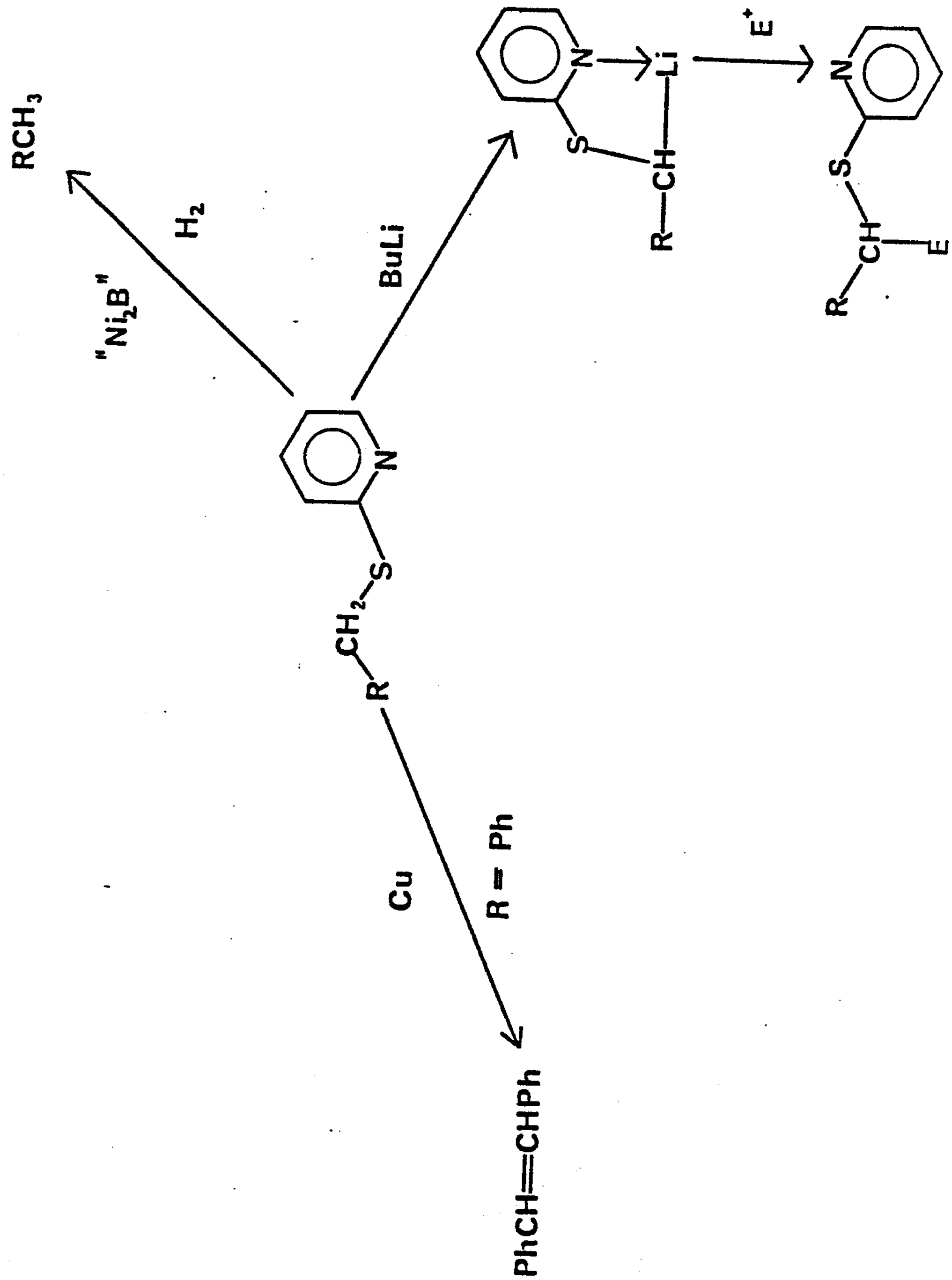
"Ni2B"
H2
BuLi
E+
Cu
R = Ph

EXEMPLES 12 à 22

Les radicaux carbonés libres R. obtenus conformément au schéma 1 réagissent avec le tri-n-butylstannane conformément au schéma 3 ci-dessous pour conduire au composé décarboxylé de formule RH :

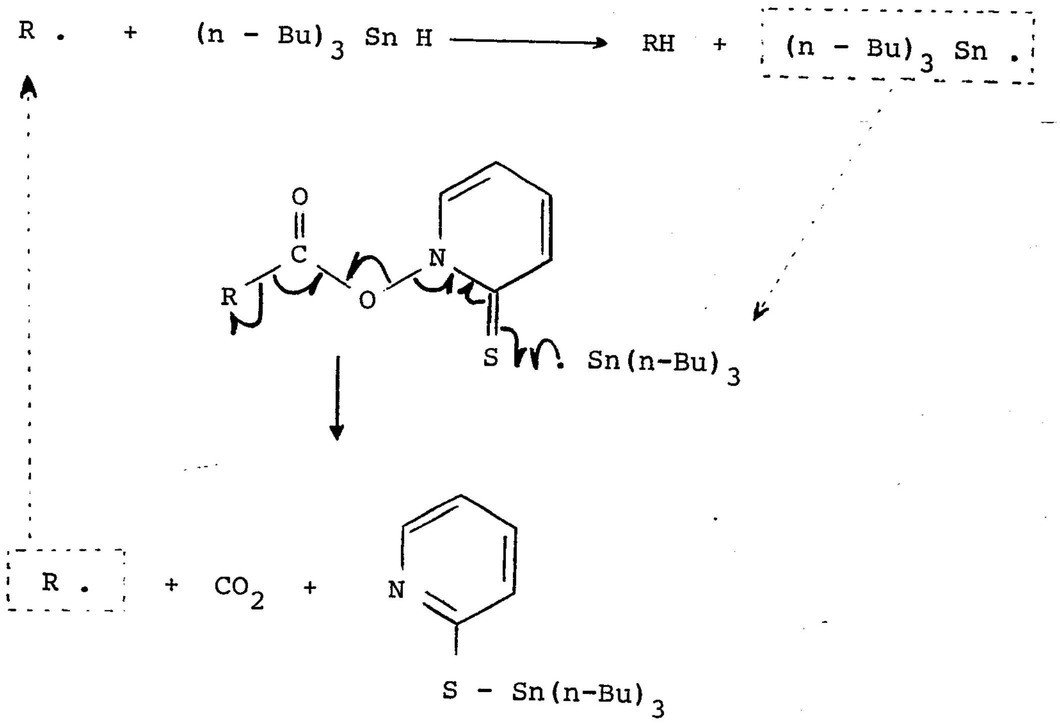

Schéma 3

Exemple 12

Préparation du n-heptadécane

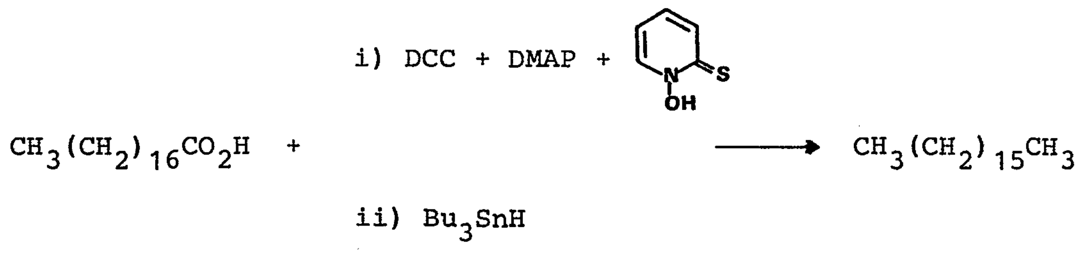


(7) (8)

286 mg (1 mmole) d'acide stéarique (7) sont mélangés avec 151 mg (1,2 mmole) de N-hydroxy-pyridine-2-thione, 183 mg (1,5 mmole) de p-diméthylaminopyridine (DMAP) et 310 mg (1,5 mmole) de dichlorohexylcarbodiimide (DCC). Le mélange dans 20 ml de benzène est agité sous une atmosphère d'azote, et la solution obtenue est portée au reflux de façon à faire distiller 10 ml de benzène. Après 45 minutes de reflux, on ajoute 10,8 ml (3 mmoles) de tri-n-butylstannane et 10 mg d'azobis-isobutyronitrile (AIBN) dans 10 ml de benzène, goutte à goutte pendant 15 minutes. Le chauffage au reflux est poursuivi pendant 6 heures, puis on ajoute 10 ml de tétrachlorure de carbone et l'on continu le reflux pendant une heure avant d'évaporer le mélange jusqu'à siccité. Le résidu est traité avec de l'iode (20 % dans 10 ml $CH_2Cl_2$) et du fluorure de potassium (10 % dans 10 ml $H_2O$), puis les deux phases sont agitées vigoureusement pendant 10 minutes. Les résidus polymères d'étain sont filtrés sous vide sur célite et lavés avec 5 ml de dichlorométhane. La phase organique est écartée, et la couche aqueuse est extraite deux fois avec du dichlorométhane (2 x 10 ml). Les phases organiques combinées sont lavées avec du

thiosulfate de sodium (10 %, 10 ml), 10 ml d'eau, séchées sur sulfate de sodium, filtrées et évaporées à siccité. Le produit brut purifié par filtration sur gel de silice (100 % pentane) donne 228 mg de n-heptadécane (8). Rendement 95 %. P.F. = 22°C.

Exemple 13

Préparation de 3β,24-diacétoxy-nor-28-oléan-12-ène

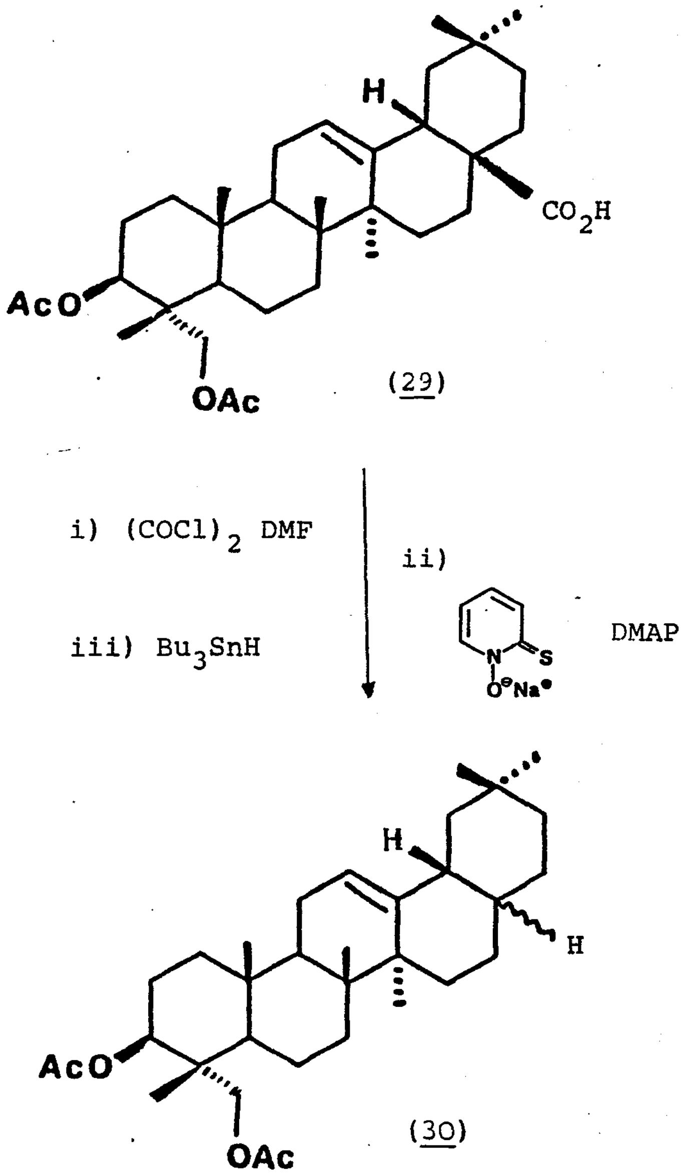


(29)

(30)

556 mg (1 mmole) de diacétate d'hédéragénine (29) sont mélangés dans 5 ml de benzène avec 1 ml de chlorure d'oxalyle. Après addition d'une goutte de DMF, l'agitation est poursuivie pendant 3 heures et le mélange est évaporé à siccité. Le résidu est repris dans 5 ml de benzène et ajouté goutte à goutte pendant 10 minutes à une suspension sous agitation de 223 mg (1,5 mmole) de sel de sodium de N-hydroxy-pyridine-2-thione et de 12 mg (0,1 mmole) de DMAP. Le mélange réactionnel est maintenu au reflux dans 10 ml de benzène sous atmosphère d'azote. Le reflux est poursuivi pendant 2 heures avant addition, goutte à goutte, pendant 10 minutes, de 2 mmoles de tri-n-butylstannane et de 10 mg d'AIBN dans 5 ml de benzène. Après 2 heures de reflux supplémentaire, 10 ml de tétrachlorure de carbone sont ajoutés et le reflux est poursuivi pendant 1 heure avant évaporation à siccité. Le résidu est traité pendant la nuit avec de l'iode (20 % dans $CH_2Cl_2$ 10 ml) et avec du fluorure de potassium (10 % dans $H_2O$ 10 ml). Les produits polymères sont filtrés sous vide. La phase aqueuse est extraite deux fois avec du dichlorométhane (2 x 10 ml) et les phases organiques combinées sont lavées avec du thiosulfate de sodium (10 % 10 ml), avec 10 ml d'eau, puis séchées sur sulfate de sodium. On filtre et on évapore à siccité. Après filtration du produit brut sur silice (éluant 75 % pentane, 25 % éther) on obtient 444 mg du composé (30). Rendement 86 %.

P.F. 114-115°C (MeOH)

$[\alpha]_D^{16}$ + 80° (C=1 dans $CHCl_3$).

$\nu$ (nujol) $cm^{-1}$ 1740, 1730.

M/Z 512 ($M^{+\cdot}$), 452 (M-60), 512   452 (M* 399,0).

$\delta$ (400 MHz dans $CDCl_3$) 0,89 (3H s) ; 0,925 (6H s) ; 0,95 (3H s) ; 1,06 (3H s) ; 1,09 (3H s) ; 2,08 (3H s) ; 2,13 (3H s) ; 2,40 (1H m) ; 3,73 (1H d, J = 8 Hz) ; 3,91 (1H d, J = 8 Hz) ; 4,81 (1H m) ; 5,13 (1H s).

$C_{33}H_{52}O_4$
trouvé : C 77,38 ; H 10,11
calculé : C 77,30 ; H 10,23.

En opérant de la même manière qu'à l'exemple 13 ci-dessus, on réalise un certain nombre de réactions du même type sur différents autres acides. Les conditions expérimentales, le rendement et la nature des produits sont consignés dans le tableau ci-dessous :

| Exemple | Acide | Température (°C) | Durée (heure) | Produit obtenu | Rendement (%) |
|---|---|---|---|---|---|
| n° 14 | (1) | 80 | 0,5 | (2) | 72 |
| n° 15 | (10) | 80 | 6 | (11) | 91 |
| n° 16 | (12) | 80 | 6 | (13) | 77 |
| | | | | (14) | 20 |
| n° 17 | (16) | 80 | 6 | (17) | 92 |
| n° 18 | (20) | 60 | 3 | (21) | 48 |
| | | | | (22) | 39 |
| n° 19 | (20) | 40 | 6 | (21) | 72 |
| | | | | (22) | 15 |
| n° 20 | (35) | 80 | 3 | (36) | 65 |
| n° 21 | (32) | 110 | 0,33 | (33) | 73 |

Remarque:

Il s'est avéré que lorsque l'on élève la température de traitement des esters dérivés des acides primaires et secondaires, on provoque une compétition entre les deux types de mécanismes réactionnels mentionnés précédemment. Ceci apparaît notamment à la lecture des exemples n° 18 et 19. Une élévation des températures conduit à une formation plus importante du dérivé de pyridyle sulfure correspondant. La formation de ce

dernier peut être réduite, voire supprimée, en abaissant la température et/ou la durée de réaction. En toute hypothèse, le pyridyle sulfure peut être aisément réduit en nor-alcane correspondant par réduction, par exemple à l'aide de nickel de Raney.

Les différents types de décarboxylation en présente de stannane sont illustrés par le schéma réactionnel ci-après :

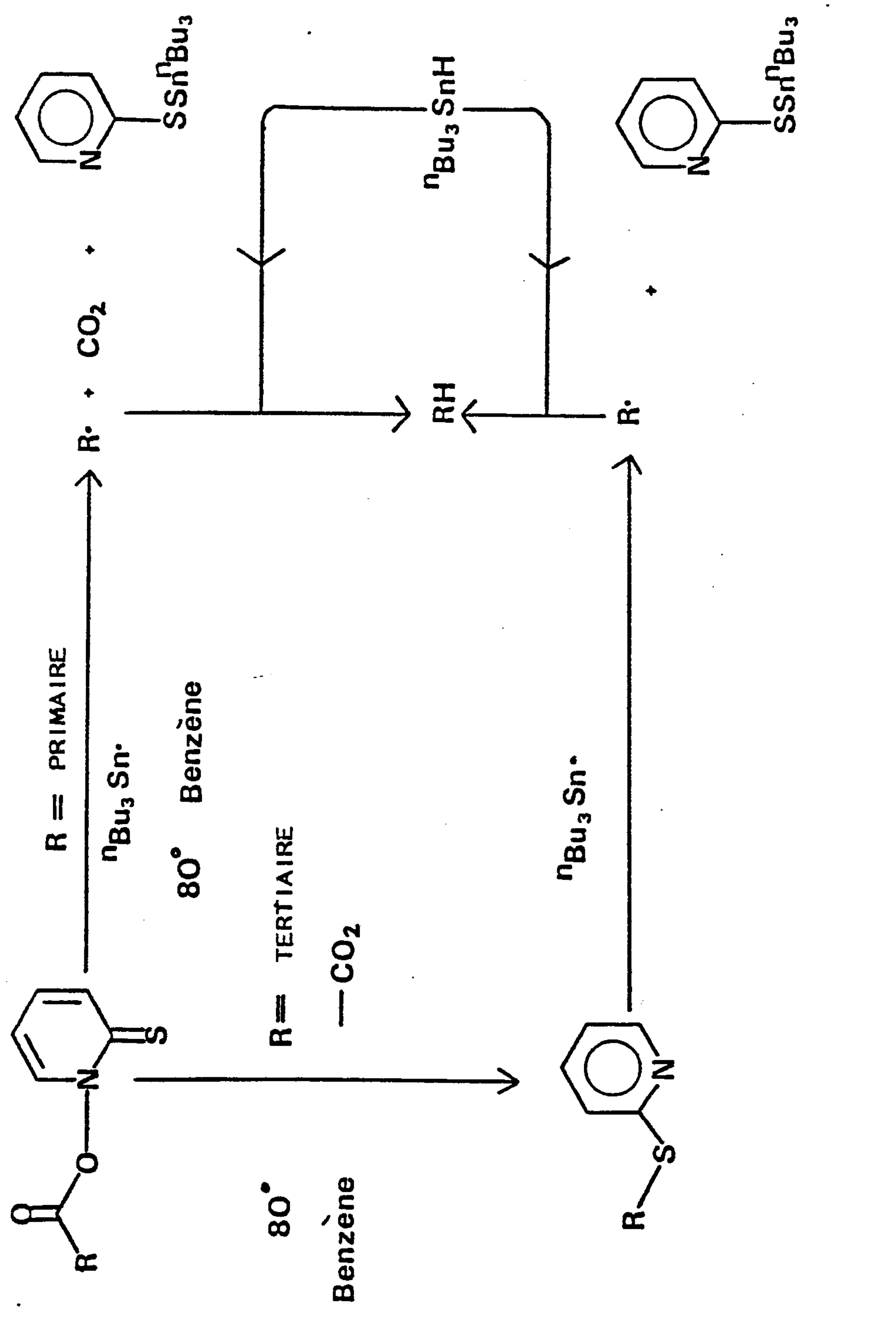


R = PRIMAIRE, SECONDAIRE , 110°, Toluène ⟶ LES DEUX PROCEDES A LA FOIS

Exemple 22

Préparation de n-heptadécane

$CH_3(CH_2)_{16}CO_2H$ + i) 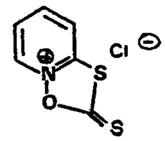

ii) $Bu_3SnH$

$CH_3(CH_2)_{15}CH_3$

(7) (8)

140 mg (1,1 mmole) de N-hydroxy-pyridine-2-thione dans 5 ml de benzène sont ajoutés goutte à goutte à température ambiante sous atmosphère d'azote à une solution de 120 mg (1,2 mmole) de phosgène dans 5 ml de benzène, ce qui conduit à une précipitation instantannée d'un solide blanc. 286 mg (1 mmole) d'acide stéarique et 0,5 ml de pyridine sont ajoutés dans 5 ml de benzène, et le milieu réactionnel est amené au reflux pendant 4 heures. 3 mmoles de tri-n-butylstannane dans 5 ml de benzène avec 10 mg de AIBN sont ajoutés au milieu de réaction durant 10 minutes et le reflux est maintenu pendant 2 heures et demie. La réaction est mise en oeuvre en présence de tétrachlorométhane, d'iode et de fluorure de potassium comme indiqué précédemment. Par filtration sur silice (100 % pentane) on obtient 168 mg de n-heptadécane. Rendement 70 %. P.F. 22°C.

Remarque: Isolation du dérivé de formule

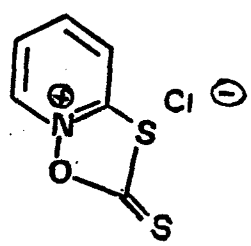

3,15 g (25 mmoles) de N-hydroxy-pyridine-2-thione dans 10 ml de benzène sont ajoutés goutte à goutte à une solution saturée de phosgène dans 40 ml de benzène durant 30 minutes à 0°C. Le précipité blanc obtenu est filtré à la trompe à eau, rincé avec une faible quantité de benzène et séché sous vide à 50°C pendant 6 heures. On obtient ainsi 4,40 g d'une poudre blanche amorphe. Rendement 98 %. P.F. 108-110°C.
$\nu$ (nujol) $cm^{-1}$ 1770.

$C_6 H_4 Cl NO_2 S$

trouvé : C 38,26 ; H 2,26 ; N 7,48 ; S 17,00 ; Cl 18,95
calculé : C 38,01 ; H 2,13 ; N 7,39 ; S 16,91 ; Cl 18,70.

## EXEMPLES 23 à 27

Les radicaux carbonés libres R. obtenus conformément au schéma 1 réagissent avec le tert-butylmercaptan conformément au schéma 4 ci-dessous pour conduire au composé décarboxylé de formule RH :

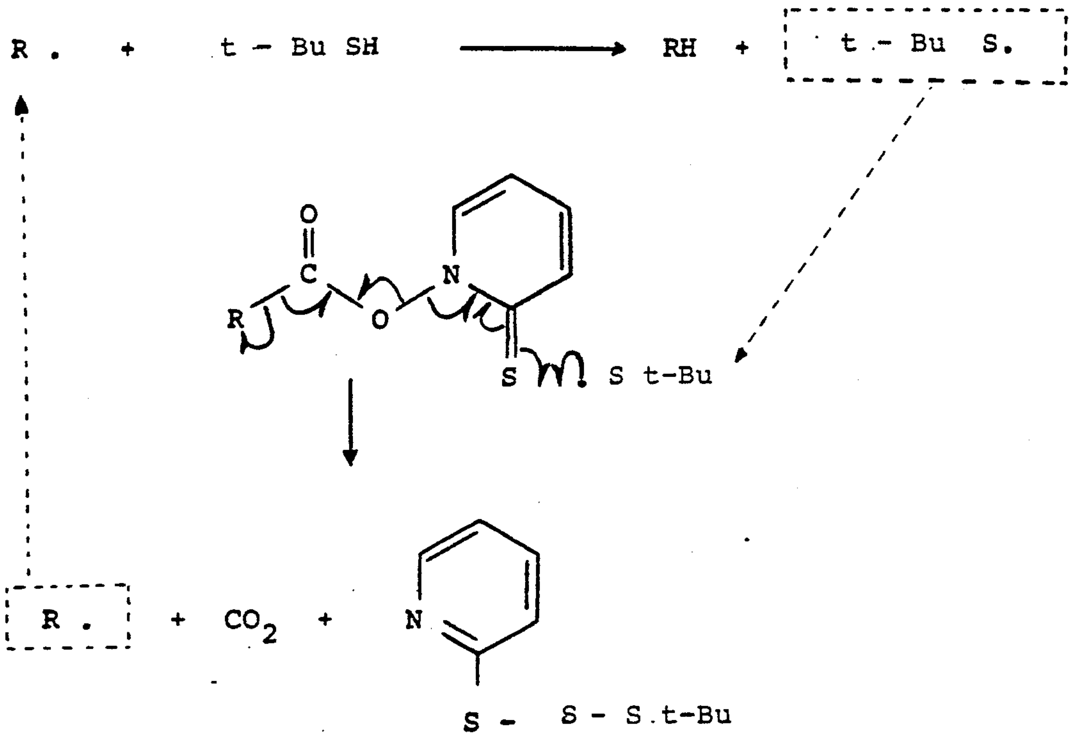


**Schéma 4-**

Exemple 23

Préparation du 3β-acétoxy-11-oxo-5α-prégnane

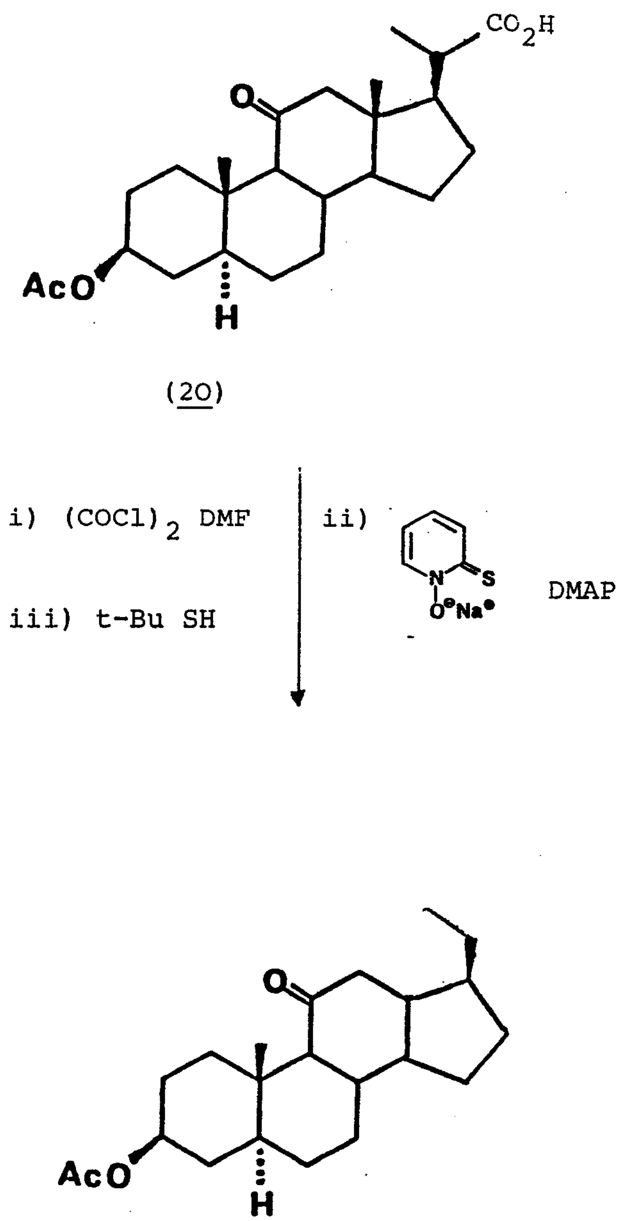


(20)

(21)

202 mg (0,5 mmole) d'acide 3β-acétoxy-bis-nor-allocholanique dans 5 ml de benzène sont traités avec 0,5 ml de chlorure d'oxalyle et 1 goutte de DMF à la température de la pièce pendant 3 heures. Après évaporation à siccité, le résidu est repris dans 5 ml de toluène et ajouté à une suspension sous agitation de 90 mg (0,55 mmole) de sel de sodium de N-hydroxy-pyridine-2-thione et de 6 mg (0,05 mmole) de DMAP, au reflux, sous atmosphère d'azote, dans 5 ml de toluène. Le reflux est maintenu pendant 1 heure avant d'être versé dans 10 ml d'une solution saturée de carbonate de potassium. La phase organique est lavée à deux reprises avec du carbonate de potassium saturé (2 x 10 ml), une fois avec de l'eau, puis avec de l'acide chlorhydrique dilué (3 x 10 ml) et finalement avec de l'eau avant séchage sur sulfate de sodium, filtration et évaporation. Le produit brut est filtré sur silice (100 % $CH_2Cl_2$) pour donner 147 mg de composé (21) se présentant sous la forme d'un solide cristallin blanc. Rendement 82 %. P.F. 160°C.

Exemple 24

En opérant comme à l'exemple 23, on réalise la réaction : (1) ⟶ (2) avec un rendement de 72 %.

Exemple 25

Préparation du 3α,12α-diacétoxy-24-norcholane

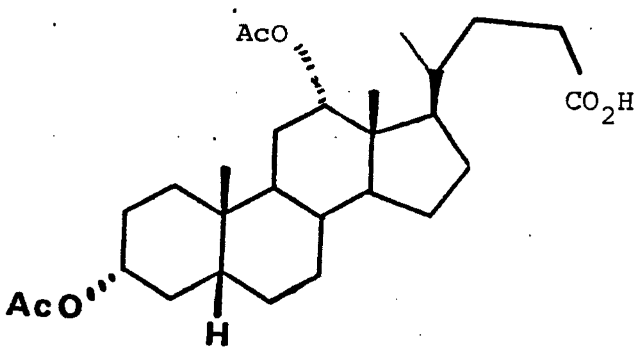

(16)

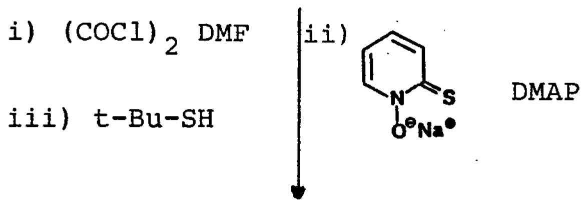


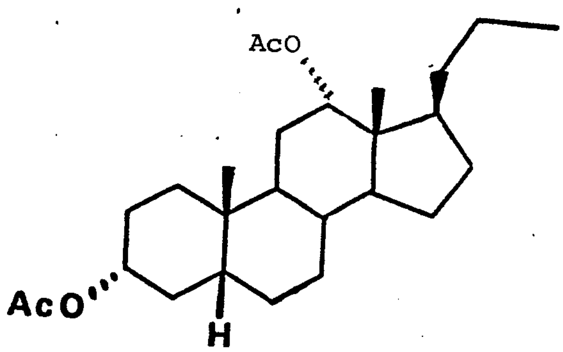

(17)

476 mg (1 mmole) d'acide diacétyldéoxycholique sont mélangés dans 5 ml de benzène avec 1 ml de chlorure d'oxalyle et 1 goutte de DMF pendant 3 heures. Après évaporation à siccité, le résidu est repris dans 5 ml de benzène et ajouté à une suspension au reflux, sous agitation, de 180 mg (1,1 mmole) de sel de sodium de N-hydroxy-pyridine-2-thione, de 12 mg (0,1 mmole) de DMAP et de 1 ml de t-butylmercaptan dans 10 ml de toluène, sous atmosphère d'azote. Le mélange est maintenu au reflux pendant 3 heures et demie, avant d'être versé dans 10 ml d'une solution saturée de carbonate de potassium. La phase organique est à nouveau lavée avec une solution saturée de carbonate de potassium (2 x 10 ml), 10 ml d'eau, deux fois 10 ml de HCl (2N) et finalement avec 10 ml d'eau avant séchage sur sulfate de sodium, filtration et évaporation à siccité. Le produit brut est filtré sur silice (100 % $CH_2Cl_2$) pour donner 321 mg du composé (17) se présentant sous la forme d'une huile incolore. Rendement 74 %. P.F. 116-117°C.

En opérant de la même manière qu'à l'exemple 25 ci-dessus, on réalise la même réaction sur deux autres acides. Les conditions expérimentales, le rendement et la nature des produits sont consignés dans le tableau ci-après :

| Exemple | Acide | Température (°C) | Durée (heure) | Produit obtenu | Rendement (%) |
|---|---|---|---|---|---|
| n° 26 | (12) | 80 | 3,5 | (13) | 62 |
| n° 27 | (29) | 80 | 3 | (30) | 85 |

EXEMPLES 28 à 40

Les radicaux carbonés libres R. obtenus conformément au schéma réactionnel 1 réagissent avec le composé $X-CCl_3$ conformément au schéma 5 ci-dessous pour conduire au composé décarboxylé de formule R-X :

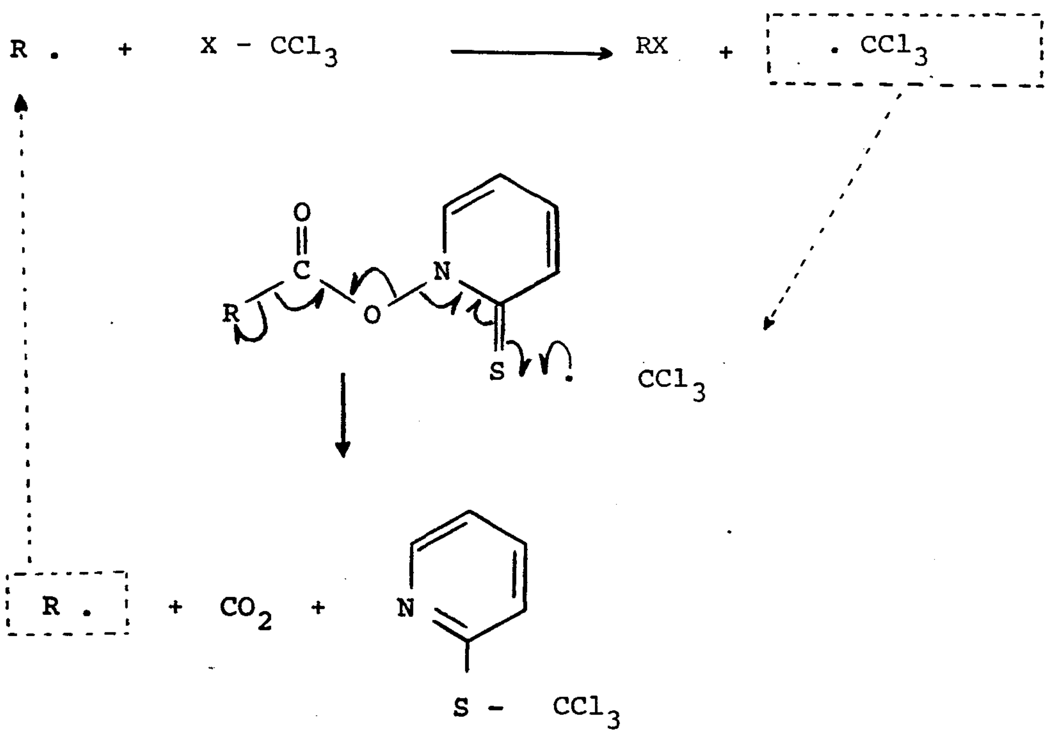

**Schéma** 5

Exemple 28

Préparation du 1-chloroadamantane

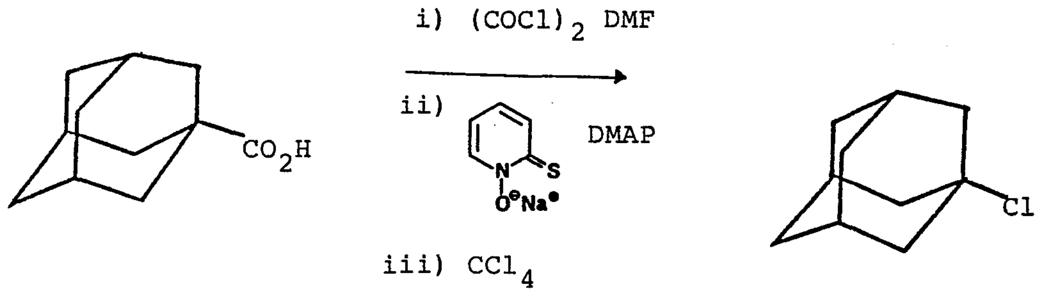


(38) (41)

180 mg (1 mmole) d'acide adamantane-1-carboxylique sont mélangés dans 5 ml de benzène avec 1 ml de chlorure d'oxalyle et 1 goutte de DMF pendant 3 heures. Après évaporation à siccité, le résidu cristallin est repris dans 5 ml de tétrachlorométhane et ajouté à une suspension au reflux de 180 mg (1,1 mmole) de sel de sodium de N-hydroxy-pyridine-2-thione et de 12 mg (0,1 mmole) de DMAP dans 10 ml de tétrachlorométhane sous atmosphère d'azote. Après 1 heure au reflux, le mélange réactionnel est refroidi, filtré et évaporé à siccité, avant de le filtrer sur silice (100 % pentane). Le produit est sublimé (100°C, 15 minutes) pour donner 150 mg de composé (41) se présentant sous la forme de cristaux incolores. Rendement 88 %. P.F. 165°C (en tube scellé).

En opérant de la même manière qu'à l'exemple 28 ci-dessus, on réalise la même réaction sur d'autres acides.

| Exemple | Acide | Chlorure obtenu | Rendement (%) |
|---|---|---|---|
| n° 29 | (1) | (4) | 72 |
| n° 30 | (16) | (19) | 95 |
| n° 31 | (23) | (26) | 72 |
| n° 32 | Acide pivalique | $(CH_3)_3$-C-Cl | 82 |

Exemple 33

Préparation de 3α-acétoxy-23-bromo-11-oxo-24-norcholane

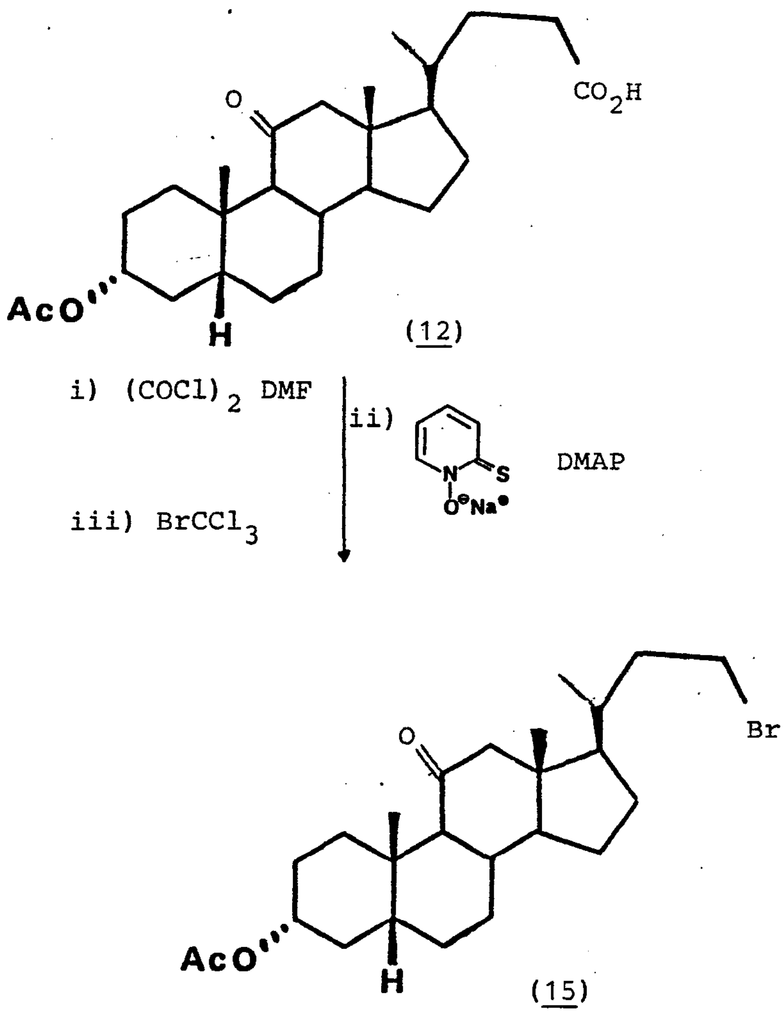


(12)

(15)

90 mg (0,2 mmole) d'acide acétyl-11-oxo-lithocholique sont mélangés dans 2 ml de benzène avec 0,25 ml de chlorure d'oxalyle et 1 goutte de DMF pendant 3 heures. Après évaporation à siccité, le résidu est repris dans 5 ml de bromotrichlorométhane et ajouté à une suspension au reflux de 50 mg (0,3 mmole) de sel de sodium de N-hydroxy-pyridine-2-thione et de DMAP (traces) dans 5 ml de bromotrichlorométhane sous atmosphère d'azote. Le reflux est maintenu pendant 1 heure et demie avant refroidissement, filtration et évaporation à siccité du mélange réactionnel. Le produit brut obtenu est filtré sur silice (100 % $CH_2Cl_2$) pour donner 75 mg du dérivé bromé de formule (15). Rendement 77 %. P.F. 164,5-165°C.

$\delta$ (60 MHz $CDCl_3$) 0,66 (3H s) ; 1,20 (3H s) ; 2,00 (3H s) ; 2,40 (3H m) ; 3,40 (2H m) ; 4,70 (1H m).

M/Z 466 ($M^{+.7}$) ; 468 ($M^{+.2}$).

$\nu$ (nujol) $cm^{-1}$ 1730, 1720.

$[\alpha]_D^{20}$ + 81° (C=0,3 M $CHCl_3$).

$C_{25}$ $H_{39}$ Br $O_3$

trouvé : C 63,97 ; H 8,35 ; Br 17,34 %

calculé : C 64,23 ; H 8,41 ; Br 17,09 %.

En opérant comme à l'exemple 33 ci-dessus, on effectue la même réaction sur d'autres acides.

| Exemple | Acide | Bromure obtenu | Rendement (%) |
|---|---|---|---|
| n° 34 | (1) | (5) | 95 |
| n° 35 | (12) | (15) | 98 |
| n° 36 | (23) | (27) | 90 |
| n° 37 | (38) | (42) | 98 |

Exemple 38

Préparation de 1,3-diphényl-2-iodopropane

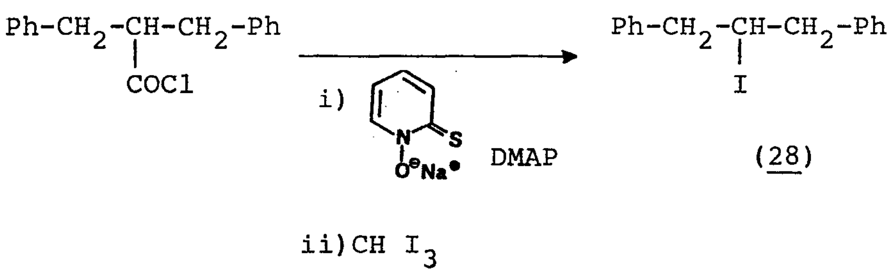


(28)

258 mg (1 mmole) de chlorure de dibenzylacétyle dans 1 ml de benzène sont ajoutés à une suspension au reflux, sous agitation, de 180 mg (1,1 mmole) de sel de sodium de N-hydroxy-pyridine-2-thione, 12 mg (0,1 mmole) de DMAP et 433 mg (1,1 mmole) d'iodoforme dans 10 ml de benzène et sous atmosphère d'azote. Le mélange réactionnel est chauffé au reflux pendant 1 heure et demie, puis refroidi, filtré et évaporé à siccité. Le produit brut est filtré sur silice (100 % pentane) pour donner 194 mg de dérivé iodé de formule (28) se présentant sous la forme d'une huile incolore. Rendement 60 %.

$\delta$ (60 MHz $CDCl_3$) 3,20 (4H d, J = 7 Hz) ; 4,38 (1H m, J = 7 Hz); 7,25 (10H s).

M/Z 322 ($M^{+.7}$)

$C_{15}H_{15}I$

trouvé : C 56,03 ; H 4,76 %

calculé : C 55,92 ; H 4,69 %.

Remarque:

En l'absence d'iodoforme, on obtient après 2 heures et demie de reflux le dérivé de pyridyle sulfure de formule (25) avec un rendement de 88 %.

De façon analogue, on obtient les dérivés iodés suivants :

| Exemple | Acide | Iodure obtenu | Rendement (%) |
|---|---|---|---|
| n° 39 | (1) | (6) | 74 |
| n° 40 | (23) | (28) | 60 |

Exemple 41

Préparation de 1,3-diphénylpronane-2-ol

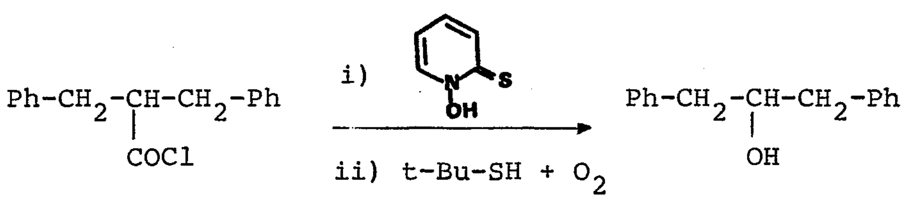


258 mg (1 mmole) de chlorure de dibenzyl-acétyle dans 1 ml de toluène sont ajoutés à la température ambiante à 140 mg (1,1 mmole) de N-hydroxy-pyridine-2-thione et 0,1 ml de pyridine dans 5 ml de toluène. Le mélange est maintenu sous agitation pendant 30 minutes. La solution filtrée est ajoutée goutte à goutte durant 20 minutes à 1 ml de t-butylmercaptan dans 20 ml de toluène à 80°C et l'on fait arriver dans cette solution un fort courant d'oxygène. La réaction est poursuivie pendant 1 heure à 80°C, puis le mélange réactionnel est agité pendant 2 heures à la température ambiante avant d'être versé dans 10 ml d'une solution saturée de carbonate de potassium. La phase organique est à nouveau lavée deux fois avec du carbonate de potassium (2 x 10 ml), puis avec 10 ml d'eau, deux fois 10 ml de HCl (2N) et finalement avec deux fois 10 ml d'eau avant séchage sur sulfate de sodium, filtration et évaporation à siccité.

Le produit brut obtenu est filtré sur silice (100 % $CH_2Cl_2$) pour donner 174 mg du dérivé hydroxylé correspondant. Rendement 82 %.

δ (60 MHz $CDCl_3$) 2,80 (4H d, J = 6 Hz) ; 4,33 (1H m, J = 6 Hz) ; 7,25 (10 s).

ν ($CH_2Cl_2$) $cm^{-1}$ 3600, 2900, 1595, 1570, 1485, 1070, 1020, 900.

M/Z 212 $M^{+.}$.

Exemple 42

En opérant de la même manière qu'à l'exemple 41, on réalise la réaction suivante :

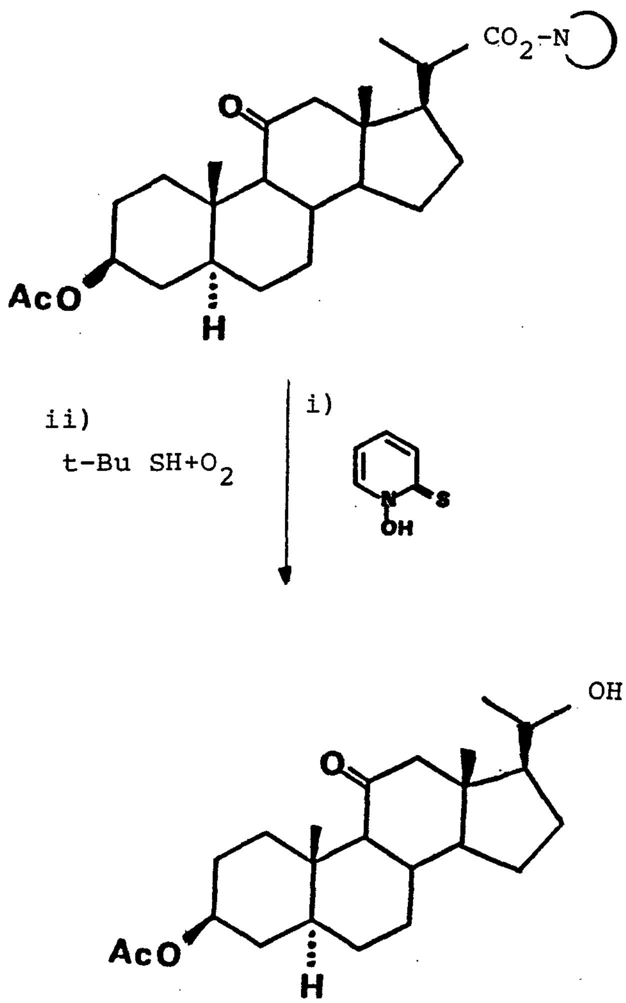

Dans ce cas particulier, on obtient le composé hydroxylé avec un rendement de 85 % en choisissant une température réactionnelle plus élevée pouvant atteindre jusqu'à environ 200°C. L'hétérocycle azoté en position 23 peut être un radical 1-pipéridyle ou 1-pyrrolidinyle. Dans cet exemple la fixation de la fonction alcool s'effectue également après formation d'un radical carboné libre résultant de la décarboxylation d'un ester thiocarbonylé de formule générale (II).

Il est clair que la présente invention ne saurait être limitée aux exemples particuliers mentionnés précédemment, mais qu'il est parfaitement possible d'en imaginer un certain nombre de variantes sans pour autant sortir du cadre de l'invention. C'est ainsi qu'il est par exemple possible d'appliquer le procédé de formation de radicaux carbonés libres R. à la réduction des anhydrides. On peut notamment conduire la réaction suivante :

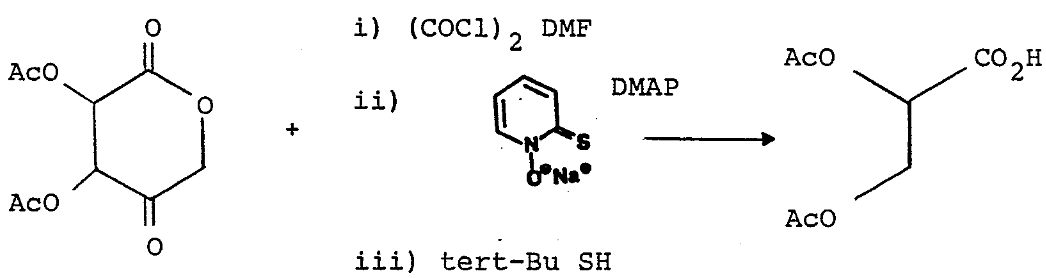


Le dérivé d'anhydride succinique ci-dessus conduit à la formation d'un acide glycérique optiquement actif utile notamment dans la synthèse de β-bloquants.

A titre d'illustration de l'application de la présente invention à la polymérisation radicalaire, on mentionnera ci-après un exemple d'homopolymérisation de l'éthylène sous haute pression en présence d'un catalyseur constitué par l'ester thiocarbonylé répondant à la formule :

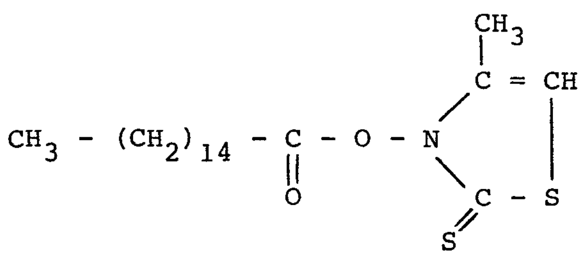

. Polymérisation de l'éthylène sous haute pression

La réaction de polymérisation de l'éthylène est conduite sous agitation dans un autoclave d'une capacité de 1 litre, dans les conditions suivantes :

- pression 1500 bars
- température 155°C
- agitation 1500 trs/min.

Les opérations ont été réalisées dans l'ordre suivant :

- mise en température du réacteur
- mise sous atmosphère d'éthylène puis introduction d'éthylène jusqu'à ce que la pression de 1500 bars soit obtenue dans l'autoclave
- stabilisation en pression et température
- injection du catalyseur - réaction
- récupération du polymère

Le catalyseur a été mis en solution dans l'heptane, à raison de 3,25 g/litre.

Cette solution a été injectée sous pression dans le réacteur, par petites quantités successives, jusqu'à obtenir un démarrage correct de la réaction. Un volume total de 19 $cm^3$ de solution catalytique a été introduit, soit 0,0647 g d'ester ou $0,165.10^{-3}$ mole d'ester.

La polymérisation a duré 15 minutes avec une élévation de température de 15°C.

En fin de réaction une quantité de 32g de polyéthylène a été recueillie, ce qui représente un taux de conversion de l'éthylène de 6,5 %.

. <u>Caractéristique du polymère obtenu</u>

L'analyse du polymère ainsi obtenu a conduit aux résultats suivants :

| | |
|---|---|
| - densité | 0,929 |
| - température de fusion | 121°C |
| - $CH_3$/1000 C | 12,9 |
| - liaison vinyl/1000 C | 0,04 |

On remarquera que le produit ainsi préparé a une densité supérieure à celle obtenue avec un peroxyde dans les mêmes conditions (0,920 - 0,924). Une meilleure cristallinité est en outre observée.

Enfin, l'exemple suivant de préparation de 2-trifluorométhylpyridine est donné en vue d'illustrer l'application de la présente invention à la perfluoration.

. <u>Schéma de réaction</u>

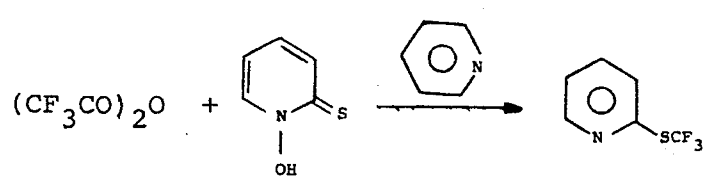

. <u>Mode opératoire</u> :

A une solution de 2-mercaptopyridine N-oxyde (0,2029g) et de pyridine (0,14 ml) dans 10 ml d'éther anhydre, on ajoute 0,25 ml d'anhydride trifluoroacétique. Le mélange est mis sous agitation à la température ambiante pendant 30 minutes sous atmosphère d'azote. La durée nécessaire pour l'estérification doit être complète.

Le mélange est ensuite irradié pendant 15 minutes à l'aide d'une lampe au tungstène.

Le sel de pyridinium formé est alors éliminé par filtration sur silice (solvant:éther) et le produit désiré est purifié par chromatographie sur colonne (solvant:éther/pentane 50/50).

0,28 g de 2-trifluorométhylthiopyridine[1] ont ainsi pu être isolé, ce qui correspond à un rendement réactionnel voisin de 100 %. Le produit a été caractérisé par spectroscopie Infra-Rouge et par RMN.

[1] Synthesis 1975/II/721-723 (L.M. YAGUPOLSKII)

REVENDICATIONS

1) Procédé de formation de radicaux carbonés libres R. éventuellement fonctionnalisés, caractérisé en ce que l'on apporte de l'énergie thermique et/ou photochimique à un ester thiocarbonylé répondant à la formule générale (I) :

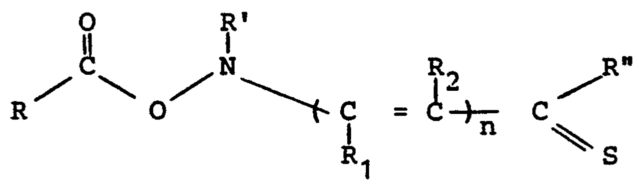

(I)

dans laquelle :

R représente un radical carboné saturé ou insaturé, linéaire ou ramifié, aliphatique ou aromatique, acyclique ou bien mono- ou poly-cyclique ou encore mono- ou poly-hétérocyclique, ledit radical carboné R étant en outre éventuellement fonctionnalisé ;

R' et R" représentent, indépendamment l'un de l'autre, un radical alcoyle, alcényle, aryle, aralcoyle, alcoylaryle, ou bien forment ensemble un hétérocycle azoté à 5 ou 6 chaînons pouvant en outre contenir un hétéroatome additionnel choisi parmi l'azote et le soufre et pouvant éventuellement être substitué ou accolé à au moins un autre cycle aliphatique ou aromatique ;

$R_1$ et $R_2$ représentent, indépendamment l'un de l'autre, un atome d'hydrogène ou un radical alcoyle inférieur, et

n est un nombre entier égal à 0, 1, 2 ou 3.

2) Procédé selon la revendication 1, caractérisé en ce que l'on porte ledit ester thiocarbonylé à une température sensiblement comprise entre 20 et 200°C, de préférence entre 70 et 120°C.

3) Procédé selon l'une des revendications 1 ou 2, caractérisé en ce que ledit ester thiocarbonylé est soumis à une irradiation de lumière visible.

4) Procédé selon l'une des revendications 1 à 3, caractérisé en ce que ledit ester thiocarbonylé est un ester thionopyridonique répondant à la formule générale (II)

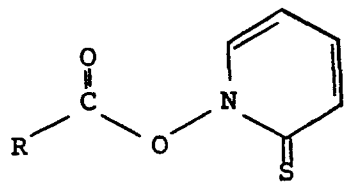

(II)

dans laquelle le radical R a la signification donnée à la revendication 1.

5) Procédé selon la revendication 4, caractérisé en ce que l'ester thionopyridonique est préparé par réaction d'un acide carboxylique libre de formule générale (III) :

$$R\ CO_2\ H \qquad (III)$$

dans laquelle R a la signification donnée à la revendication 1, avec un composé de formule (IV)

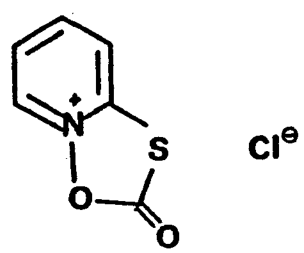

(IV)

ce dernier étant obtenu par réaction du phosgène sur la N-hydroxy-pyridine-2-thione.

6) Procédé selon la revendication 4, caractérisé en ce que l'ester thionopyridonique est préparé par réaction de l'anhydride mixte de formule générale (V) :

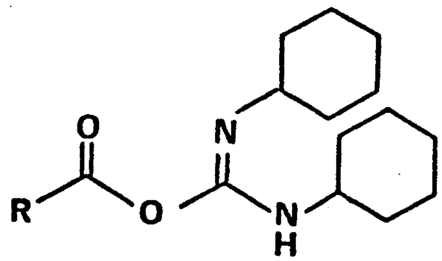

(V)

dans laquelle R a la signification donnée à la revendication 1, avec la N-hydroxy-pyridine-2-thione en présence d'un catalyseur tel que la p-diméthylaminopyridine.

7) Procédé selon la revendication 4, caractérisé en ce que l'ester thionopyridonique est préparé par réaction d'un chlorure d'acide de formule générale (VI)

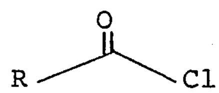

dans laquelle R a la signification donnée à la revendication 1, avec le sel de sodium de la N-hydroxy-pyridine-2-thione en présence d'un catalyseur tel que la p-diméthylaminopyridine.

8) Procédé de préparation d'un composé de formule générale R-X à partir d'un acide carboxylique de formule générale $R-CO_2H$, dans lesquelles R a la signification donnée à la revendication 1, caractérisé en ce que l'on ajoute au milieu réactionnel de formation de radicaux libres carbonés R. un composé de formule générale X-Y dans laquelle X, qui représente le groupe à greffer sur le radical libre carboné R., est choisi parmi les atomes d'hydrogène, de chlore, de brome et d'iode, etc., et Y représente un groupe porteur de chaîne choisi parmi $n-Bu_3\ S^{\cdot}$, tert-Bu $S^{\cdot}$, $CCl_3^{\cdot}$, $CHI_2^{\cdot}$, $R_3Sn^{\cdot}$, $ArSO_2^{\cdot}$.

9) Procédé de préparation d'un composé de formule générale R-A-B-X à partir d'un acide carboxylique de formule générale $R-CO_2H$, dans lesquelles R a la signification donnée à la revendication 1, caractérisé en ce que, en plus du composé de formule X-Y défini à la revendication 8, on ajoute au milieu réactionnel de formation de radicaux carbonés libres R. un composé de formule générale A=B choisi parmi l'oxygène, les composés à insaturation éthylénique et les dérivés azoïques.

10) Application du procédé de formation de radicaux carbonés libres R. selon l'une des revendications 1 à 9 à la polymérisation radicalaire, en particulier la polymérisation de monomères alcéniques.

0141690

Office européen des brevets

# RAPPORT DE RECHERCHE EUROPEENNE

Numéro de la demande

EP 84 40 1697

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | CHEMICAL ABSTRACTS, volume 52, no. 1, 10 janvier 1958, colonne 284c, (COLUMBUS, OHIO, US); L. HORNER et al.: "Course of substitutions. XII. Rearrangement and pyrolytic degradation of acylated hydroxylamines", & Ann. 606, 24-47(1957), c.f. C.A. 51, 12879b<br>----- | 1,2 | C 07 B 61/02<br>C 08 F 10/00<br>C 08 F 4/32 //<br>C 07 C 1/253<br>C 07 C 9/22<br>C 07 C 15/12<br>C 07 C 17/33<br>C 07 C 19/02<br>C 07 C 21/24<br>C 07 C 23/28<br>C 07 C 29/00<br>C 07 C 33/24<br>C 07 D 213/70<br>C 07 J 7/00<br>C 07 J 9/00<br>C 07 J 43/00 |

DOMAINES TECHNIQUES RECHERCHES (Int. Cl.4)

C 07 B 61/00
C 07 C 1/00
C 07 C 9/00
C 07 C 17/00
C 07 D 213/00

Le présent rapport de recherche a été établi pour toutes les revendications

| Lieu de la recherche | Date d'achèvement de la recherche | Examinateur |
|---|---|---|
| LA HAYE | 28-11-1984 | WRIGHT M.W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82